# EUROPEAN PATENT APPLICATION

(11) **EP 0 911 034 A1**
(43) Date of publication of application: **28.04.1999**
(21) Application number: 98901522.7
(22) Date of filing: 05.02.1998
(51) Int. Cl.: A61K 38/22, A61K 45/00

(54) **MEDICINAL COMPOSITIONS FOR TREATING CARDIAC DISEASES CAUSED BY CARDIAC HYPERTROPHY**

(30) Priority: 05.02.1997 JP 22594/97
(71) Applicant: SUNTORY LIMITED, Osaka-shi, Osaka 530-0004 (JP)
(72) Inventor: INOMATA, Norio, Suntory Inst.for Biomedical Res., Mishima-gun, Osaka 618-8503 (JP); YAMAKI, Akira, Suntory Inst. for Biomedical Res., Mishima-gun, Osaka 618-8503 (JP); FURUYA, Mayumi, Suntory Inst. for Biomedical Res., Mishima-gun, Osaka 618-8503 (JP); HIDAKA, Toshinori, Suntory Inst. for Biomedical, Mishima-gun, Osaka 618-8503 (JP)
(74) Representative: Stoner, Gerard Patrick
(86) International application number: JP9800483
(87) International publication number: WO9834636

(57) **Abstract**

The present invention provides a pharmaceutical composition for treatment of heart disease based on cardiac hypertrophy, a cause of the onset of heart diseases such as ischemic heart disease and arrhythmia. Consequently, the present invention provides a pharmaceutical composition for treatment of heart disease based on cardiac hypertrophy having for its active ingredient a substance that binds to the NP receptor, GC-A, and is able to accelerate the production of cGMP. Specific examples of active ingredients include natriuretic peptides such as atrial natriuretic peptide and brain natriuretic peptide.

## Description

### Technical Field

The present invention relates to a pharmaceutical composition for treatment of heart disease based on cardiac hypertrophy comprising as its active ingredient a substance that acts on the natriuretic peptide (NP) receptor, guanylyl cyclase A (GC-A), and is able to stimulate production of cyclic guanosine monophosphate (cGMP).

### Background Art

Following the occurrence of increased load on the ventricle due to hypertension or valvular heart disease, or disorders of cardiac myocytes themselves such as myocardial infarction, myocarditis and cardiomyopathy, the shape of the ventricle is altered primarily by hypertrophy of heart muscle cells so as to maintain cardiac output resulting in the so-called state of cardiac hypertrophy. Although this cardiac hypertrophy is considered to be an adaptation phenomenon in response to cardiac myoctes disorders and mechanical load to a certain extent, when hypertrophy becomes pronounced due to continuous application of an excess load, contraction and dilation functions are hindered. In addition to leading to decreased cardiac output and chronic heart failure, there is an increased risk of falling into an ischemic state and greater susceptibility to potentially fatal arrhythmia. The degree of cardiac hypertrophy is considered to be one of the factors that determines the prognosis of heart disease. According to large-scale progressive surveys as represented by the Framingham Study, the presence of cardiac hypertrophy initiates the onset of heart failure, which in turn has been determined to increase the risk of angina pectoyis, myocardial infarction and other ischemic heart diseases as well as arrhythmia by 2.5 to 3 times (Yamazaki, R. and Yazaki, Y., Heart failure, p. 37-45, Shinoyama, S. ed., Pharmaceutical Journal Publishing, 1997). Thus, a drug that inhibits the excessive formation of cardiac hypertrophy or reduces the hypertrophy is considered to be effective in preventing the onset and progress of heart diseases including chronic heart failure.

Treatment of chronic heart failure has conventionally consisted primarily of the use of cardio tonics for the purpose of improving heart contraction and increasing cardiac output. However, although cardio tonics exhibit immediate effects that improve subjective symptoms and exercise tolerance, they lack effects in terms of improving survival prognosis, namely a life-extending effect, which is the ultimate objective of treatment of chronic heart failure. On the contrary, results have been obtained indicating that they may actually result in a worse prognosis (Packer, et al., N. Engl. J. Med., Vol. 325, p. 1468, 1991).

On the other hand, catecholamines, angiotensin II, aldosterone, endothelin, vasopressin and other neurohumoral factors have been suggested to be involved in the occurrence and progress of cardiac hypertrophy, and development and clinical application has progressed on drugs that inhibit the production or signal transduction system of these factors. However, since a plurality of these factors are involved in the mechanism of occurrence of cardiac hypertrophy in the body, a drug that exhibits antagonistic effects on only one of these factors is expected to be insufficient. For example, although an inhibitor of angiotensin conversion enzyme (ACE), an enzyme involved in the production of angiotensin II, has been observed to inhibit the occurrence and progress of cardiac hypertrophy in animal models (Brilla, et al., Circulation, Vol. 83, p. 1771, 1991), and in the case of clinical administration to chronic heart failure patients, extends the prognosis for survival by reducing cardiac hypertrophy (The Save Investigation, N. Engl. J. Med., Vol. 327, p. 678, 1992), its effects have as of yet not been considered to be sufficient.

In actuality, even if the most advanced treatment is performed, the mortality rate at five years after onset of chronic heart failure is presently about 50%. Although endothelin antagonists (Ito, et al., Circulation, Vol. 89, p. 2198, 1994) and vasopressin antagonists (Tomura, et al., Circulation, Vol. 94 (Suppl. I-264), 1996) have recently been reported to inhibit the formation of cardiac hypertrophy in animal models, there is a need to develop drugs for the treatment of heart disease that inhibit cardiac hypertrophy based on a new mechanism. Disclosure of the Invention

Thus, the object of the present invention is to provide a pharmaceutical composition effective in preventing cardiac hypertrophy involved in the onset and progress of chronic heart failure and other heart diseases, and more particularly, to provide a pharmaceutical composition for treatment of heart disease based on cardiac hypertrophy comprising as its active ingredient a substance able to stimulate cGMP production by binding to GC-A, a natriuretic peptide (NP) receptor.

### Brief Description of the Drawings

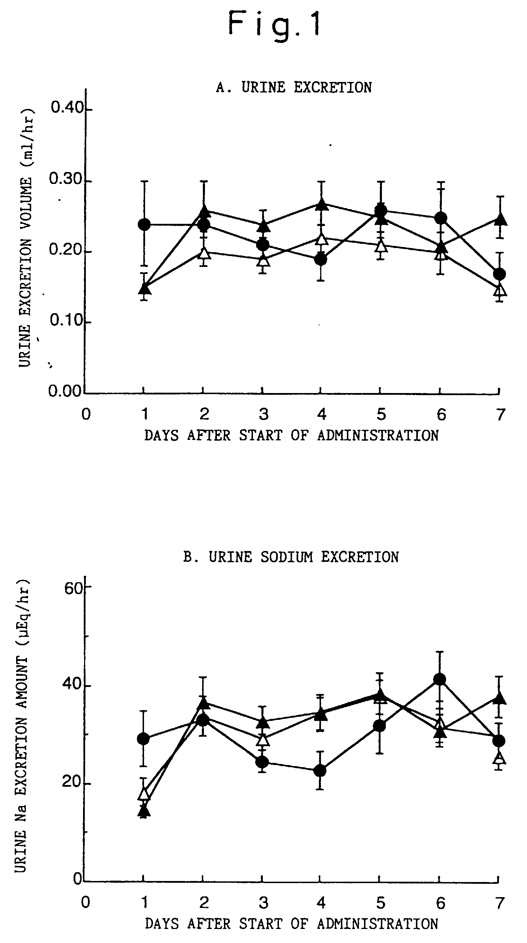

Fig. 1 is a graph showing the time-based changes in urine excretion volume (A) and urine sodium excretion amount (B) in a sham group, control group and ANP group in the case of administering 0.1 µg/kg/min of ANP or 5% glucose for 1 week starting immediately after the operation in a rat model of hypertrophy induced by aortic banding. △ indicates the sham group (n = 19), ▲ indicates the control group (n = 20) and ● indicates the ANP group (n = 12). In addition, each value represents the mean ± standard error.

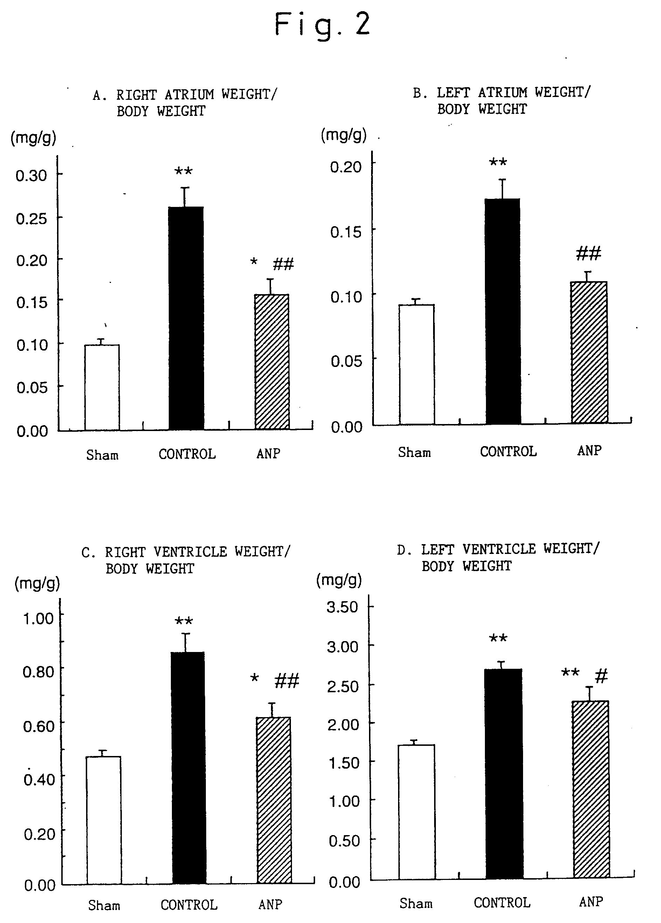

Fig. 2 is a graph showing the right atrium weight/body weight ratio (A), left atrium weight/body weight ratio (B), right ventricle weight/body weight ratio (C) and left ventricle weight/body weight ratio (D) in a Sham group, control group and ANP group in the case of administering 0.1 µg/kg/min of ANP or 5% glucose for 2 weeks starting two weeks after surgery in a rat model of cardiac hypertrophy induced by arteriovenous shunt of the abdominal aorta. In addition, * and ** indicate the presence of a significant difference from the sham group at p<0.05 and p<0.01, respectively, while # and ## indicate the presence of a significant difference from the control group at p<0.05 and p<0.01, respectively. The results were tested for the presence of a significant difference by ANOVA analysis.

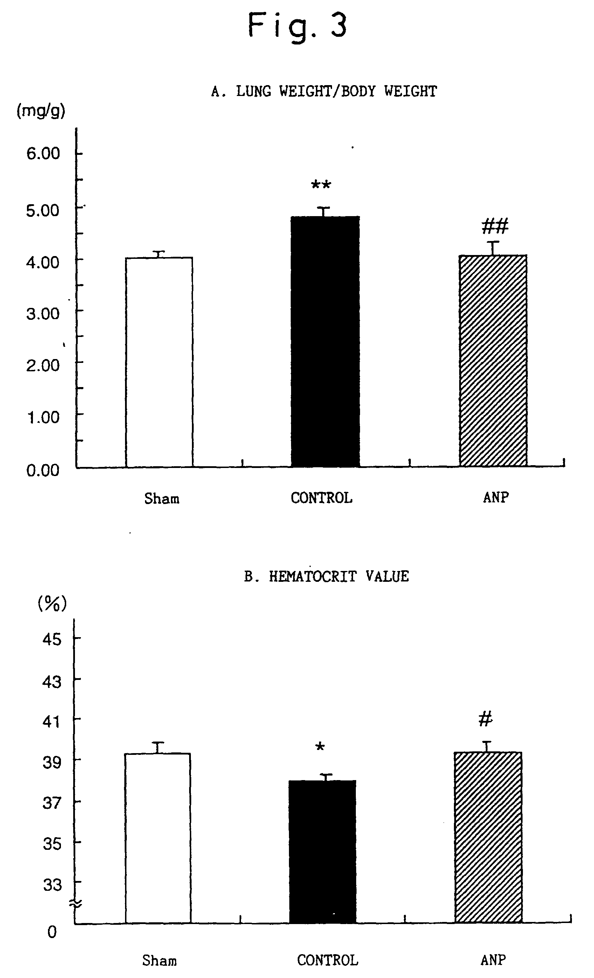

Fig. 3 is a graph showing the lung weight/body weight ratio (A) and hematocrit value (B) of a sham group, control group and ANP group at the end of the study under the same conditions as in Fig. 2. Each value represents the mean ± standard error of 6 animals. In addition, * and ** indicate the presence of a significant difference from the sham group at p<0.05 and p<0.01, respectively, while # and ## indicate the presence of a significant difference from the control group at p<0.05 and p<0.01, respectively. The results were tested for the presence of a significant difference by ANOVA analysis.

### Embodiment for Carrying Out the Invention

The present invention relates to a pharmaceutical composition for treatment of heart disease based on cardiac hypertrophy comprising as its active ingredient a substance that acts on the natriuretic peptide (NP) receptor, guanylyl cyclase A (GC-A), and is able to stimulate production of cyclic guanosine monophosphate (cGMP). Specific examples of heart diseases based on cardiac hypertrophy include heart failure (e.g., chronic heart failure), ischemic heart disease (e.g., myocardial infarction and angina pectoris) and arrhythmia.

There are no particular limitations on substances that can be used as the active ingredient of the pharmaceutical composition as claimed by the present invention provided that they have the property of being able to stimulate production of cGMP by way of GC-A, an NP receptor. Substances having said property can be obtained (1) in the form of a substance that is able to increase said activity by measuring intracellular cGMP concentration, or (2) in the form, of a substance that is able to increase said activity by measuring guanylyl cyclase activity in cell or tissue membrane fractions that express GC-A, when the candidate substance is added to cells or tissue that expresses GC-A (for example, vascular endothelial cells, vascular smooth muscle cells, adrenal cortex zona glomerular cells, cells in which GC-A is artificially expressed, or the aorta, pulmonary artery, glomerulus and adrenal cortex) followed by incubation. Furthermore, known methods can be used for the above-mentioned measurement methods, examples of which are described in, for example, Minamitake, Y., et al., Biochem. Biophys. Res. Commun., 172, 971-978 (1990), Furuya, M., et al., Biochem. Biophys. Res. Commun., 177, 927-931 (1991), and Hidaka, Y., et al., Jap. J. Pharm., 101, 309-325 (1993). Preferable examples of substances having the above-mentioned property include natriuretic peptide (NP), more preferably atrial natriuretic peptide (ANP) and brain natriuretic peptide (BNP).

Moreover, although human α-hANP consisting of 28 amino acids (Kangawa, et al., Biochem. Biophys. Res. Commun., Vol. 118, p. 131, 1984) (SEQ ID NO: 1) or rat α-rANP (Kangawa, et al., Biochem. Biophys. Res. Commun., Vol. 121, p. 585, 1984) (SEQ ID NO: 2) can be used as the ANP, the peptide of the active ingredient according to the present invention may be any peptide having an ANP ring structure (formation of a disulfide bond based on Cys residues) and a C terminal portions continuing from a ring structure. An example of said peptide is the peptide having the amino acid residues at positions 7 through 28 of α-hANP (SEQ ID NO: 3). Human α-hANP is particularly preferable as the ANP.

In addition, an example of BNP is human BNP consisting of 32 amino acids (Sudoh, et al., Biochem. Biophys. Res. Commun., Vol. 159, p. 1420, 1989) (SEQ ID NO: 4).

Based on the amino acid sequence of the above-mentioned substance (e.g., α-hANP), a person with ordinary skill in the art could perform modification in the form of deletion, substitution, addition or insertion on amino acid residues in said sequence using suitable known methods, and any of the resulting peptides can be used provided the resulting peptide acts on the ANP receptor, GC-A, and is able to stimulate production of cGMP.

In addition, peptides that have been isolated and purified from natural substances, as well as peptides produced by chemical synthesis or genetic recombination can also be used as said peptide. In addition to those previously mentioned, examples of these peptides include frog ANP (SEQ ID NO: 5), pig NP (SEQ ID NO: 6), rat BNP (SEQ ID NO: 7) and chicken BNP (SEQ ID NO: 8).

Moreover, the substance pertaining to the active ingredient of the pharmaceutical composition of the present invention can also be used in the form of an acid addition salt of an inorganic acid such as hydrochloric acid, sulphonic acid and phosphoric acid, or of an organic acid such as formic acid, acetic acid, butyric acid, succinic acid and citric acid. The above-mentioned substance may also be in the form of a salt resulting from a metal salt of sodium, potassium, lithium or calcium, etc., or from an organic base. In addition, the pharmaceutical composition as claimed in the present invention may be in the free form of the substance pertaining to its active ingredient or in the form of its pharmacologically acceptable salt.

The substance according to the present invention or its pharmacologically allowable salt is preferably administered by an administration method typically used for pharmaceuticals, namely oral administration or non-oral administration such as intravenous administration, intramuscular administration or subcutaneous administration, after mixing with a pharmacologically acceptable carrier, vehicle, diluent or so forth which is itself known.

In the case the active ingredient is a peptide, since the peptide is subjected to breakdown in the digestive organs with oral administration, this administration method is typically not effective. However, it is also possible to administer the peptide in the form of a preparation that is resistant to breakdown in the digestive organs, for example, a microcapsule preparation in which the active ingredient in the form of a peptide is contained in a ribosome. In addition, peptides can also be administered by allowing them to be absorbed from a mucous membrane other than the digestive organs such as the rectum, within the nose and under the tongue. In this case, they can be administered in the form of a suppository, nasal spray or sublingual lozenge.

Although the dose of the pharmaceutical composition as claimed in the present invention varies according to the type of disease, patient's age, body weight and severity of symptoms, and the administration route, it can typically be administered over a range of 0.1 µg/kg to 100 mg/kg, and preferably over a range of 0.5 µg/kg to 5 mg/kg.

ANP is a peptide hormone that is secreted by the heart and plays an important role in regulating water electrolyte metabolism and blood pressure. ANP receptors employ a membrane bonding type of guanylyl cyclase structure, and are referred to as GC-A or NPR-A. Namely, ANP bonds to GC-A and increases intracellular levels of cGMP resulting in physiological effects such as diuresis and vasodilation.

In humans and model animals, plasma ANP concentration is known to rise depending on the degree of severity of cardiac hypertrophy and heart failure. In addition, although ANP is primarily synthesized in the atria in the normal heart, production of ANP in the ventricles increases remarkably during cardiac hypertrophy. In this manner, ANP, the production of which is accelerated as the disease state progresses, is considered to act defensively in the form of a compensatory mechanism in response to heart failure. In actuality, vasodilatory and diuretic effects are exhibited following short-term (1-24 hours) administration of ANP in acute heart failure patients, and ANP has been observed to demonstrate effects that relieve heart preload and afterload, resulted in the improvement of hemodynamics.

However, whether or not ANP has any effects on cardiac hypertrophy has yet to be determined. On the other hand, although Cao, et al. reported that ANP inhibits DNA synthesis of cultured cardiac fibroblasts in vitro (Cao, et al., Hypertension, Vol. 25, p. 227, 1995), the effect of ANP on hypertrophy of cardiac myocytes, which account for the largest volume in the ventricles (approx. 70%) and play a central role in cardiac hypertrophy, has yet to be studied.

Moreover, although diuretic effects and mild inhibitory effects on cardiac hypertrophy have been reported to be demonstrated by the inhibition of neutral endopeptidase (NEP, EC 3.4.24.11), which is involved in the metabolism and clearance of ANP in the body, in rat model of cardia hypertrophy volume loading, increases in blood ANP concentration at this time are not clear. In addition, since NEP is not only involved in ANP metabolism, but also in the metabolism of other peptide hormones such as bradykinin, it is not clear as to whether diuretic effect and inhibitory effect on cardiac phypertrophy were actually based on inhibition of degradation of ANP, namely whether they actually reflected the effects of ANP (Willenbrock, et al., Hypertension, Vol. 27, p. 1259, 1996).

In this manner, it cannot be presently determined whether or not the occurrence of cardiac hypertrophy is inhibited or whether or not cardiac hypertrophy is reduced by administration of ANP itself.

Thus, the inventors of the present invention examined the preventive and reducing effects of continuous administration of ANP on cardiac hypertrophy according to the method described hereinafter using a model of aradiac hypertrophy induced by pressure-overload.

In addition, cardiac hypertrophy is induced by various factors such as increased pressure load due to high blood pressure and increased volume load due to valvular heart diseased. Therefore, in order to further clarify the cardiac hypertrophy inhibitory effects of ANP on cardiac hypertrophy, in addition to the model of hypertensive pressure-load described above, another model of induced by cardiac hypertrophy due to volume-load abdominal arteriovenous shunt was prepared according to the method described below. Moreover, since this model also exhibits pulmonary congestion, a major symptom of chronic heart failure, the effect of ANP on pulmonary congestion was also examined.

### A. Experimental Method for Investigating Preventive and Reducing Effects on Cardiac Hypertrophy Using a Model of Hypertrophy Induced by Pressure-Overload

### 1. Preparation of Rat Model of Cardiac Hypertrophy by Pressure Overload

Nine-weeks-old, male Sprague-Dawley rats were used in the experiment. The animals were anesthetized by intraperitoneal administration of pentobarbital sodium (40 mg/kg) and then placed in the prone position. Following laparotomy, the abdominal aorta was exposed and the portion between the left and right renal arteries was detached. A 21G needle was aligned in the aorta and ligated with silk thread between the left and right renal arteries together with the aorta. Next, the aorta was constricted by pulling out the needle. In this model, systolic blood pressure increases due to constriction of the abdominal aorta in this manner, and this results in increased cardiac afterload leading to the occurrence of left ventricle hypertrophy. Only detachment of the abdominal aorta was performed on animals of a sham operation (sham) group.

### 2. ANP Administration Method

An incision was made into the necks of rats under anesthesia induced by intraperitoneal administration of pentobarbital sodium (40 mg/kg) followed by the insertion of a silicon catheter into the right jugular vein. The catheter was lead subcutaneously to cervical dosal region, and was connected to a microinfusion pump. Human ANP (α-hANP) was dissolved in 5% glucose and administered by intravenous infusion at the rate of 0.1 µg/kg/min. 5% glucose was administered by intravenous infusion at the rate of 2.5 µl/min to animals of the control and sham groups.

### 3. Evaluation Method

The rats were anesthetized by intraperitoneal administration of pentobarbital sodium (40 mg/kg) at the end of the study followed by the insertion of a polyethylene catheter into the left carotid artery and measurement of blood pressure and heart rate by a pressure transducer. Following measurement, 2 ml of blood was collected from the carotid artery using a syringe containing 1/10 volume of 1% (w/v) EDTA 2Na and 5000 KIU/ml of aprotinin in order to measure plasma α-hANP and rat ANP concentrations as well as cGMP concentration, a second messenger of intracellular signal transduction of ANP. Blood samples were immediately separated by centrifugation at 4°C, and the resulting plasma was stored at -80°C. Plasma α-hANP, rat ANP and cGMP were measured by radioimmunoassay using anti-α-hANP rabbit serum, anti-rat ANP rabbit serum and anti-succinyl cGMP monoclonal antibody respectively. Moreover, following blood sampling, an excess amount of pentobarbital sodium was administered intravenously to sacrifice the rats. After measuring body weight, the hearts of the animals were exased and separated into the left ventricle and right ventricle followed by measurement of their respective weights. The ratio of left ventricle weight to body weight was then used as an indicator of cardiac hypertrophy.

### B. Experimental Method for Investigating Inhibitory Effects on Cardiac Hypertrophy and Pulmonary Congestion Ameriloration Using a Model of Cardiac Hypertrophy by Volume Overload

### 1. Preparation of Rat Model of Cardiac Hypertrophy by Volume Loading

Nine-weeks-old, male Sprague-Dawley rats were used in the experiment. The animals were anesthetized by intraperitoneal administration of pentobarbital sodium (40 mg/kg) and then placed in the prone position. Following laparotomy, the abdominal aorta and vena cava were exposed and clamps were placed at the renal artery branch and femoral artery branch of the aorta to interrupt blood flow. An 18G needle was inserted into the aorta at the clamped portions, and advanced into the adjacent interior vena cava to prepare an arteriovenous shunt. The needle was withdrawn, the aortic purcuture site was sealed with surgical adhesive, and the clamps were removed. After confirming the flow of arterial blood into the vena cava at the shunt portion, the abdomen was closed. In this model, venous pressure increased due to the formation of this abdominal aorta-vena cava shunt resulting in increased cardiac preload. Loads were applied to the right atrium, right ventricle, left atrium and left ventricle in that order leading to the formation of hypertrophy. Moreover, due to the low compliance of the venous system, blood began to pool resulting in the occurrence of pulmonary congestion. Only detachment of the abdominal aorta and vena cava was performed on animals of a sham operation (sham) group.

### 2. ANP Administration Method

ANP was administered in the same manner as in the case of the pressure-loaded cardiac hypertrophy model.

### 3. Evaluation Method

The rats were anesthetized by intraperitoneal administration of pentobarbital sodium (40 mg/kg) at completion of the study followed by the insertion of a polyethylene catheter into the left carotid artery and left jugular vein and measurement of blood pressure, heart rate and right atrial pressure. Following measurement, 2 ml of blood were collected from the carotid artery using a syringe containing 1/10 volume of 1% (w/v) EDTA 2Na and 5000 KIU/ml of aprotinin in order to measure plasma α-hANP and rat ANP concentrations. Blood samples were immediately separated by centrifugation at 4°C, and the resulting plasma was stored at -80°C. Plasma α-hANP and rat ANP concentrations were measured by radioimmunoassay using anti-α-hANP rabbit serum and anti-rat ANP rabbit serum respectively. In addition, arterial blood was collected in a hematocrit tube and centrifuged followed by determination of hematocrit value. Moreover, following blood sampling, an excess amount of pentobarbital sodium was administered intravenously to sacrifice the rats. After measuring body weight, the hearts of the animals were excised and separated into the right atrium, left atrium, right ventricle, left ventricle and lungs followed by measurement of their respective weights. The ratios of each heart chamber and lungs to body weight was then used as an indicator of cardiac hypertrophy and pulmonary congestion.

### Examples

The following provides a detailed explanation of the present invention through Examples.

### Example 1. Study of the Preventive Effects of ANP on Cardiac Hypertrophy

The inventors of the present invention first examined the cardiac hypertrophy preventive effects of ANP. A silicon catheter was inserted into the jugular vein immediately after performing abdominal aorta constriction surgery or sham surgery on rats, followed by continuous intravenous infusion of α-hANP at 0.1 µg/kg/min or 5% glucose at 2.5 µl/min for 1 week. One week later, hemodynamic parameters of the rats were measured under anesthesia, and the weight of the heart was measured after sacrificing the animals following collection of blood samples. Those results are shown in Table 1.

**Table 1**

| Effects of Continuous Intravenous Infusion of ANP for 1 Week on Body Weight, Heart Weight, Blood Pressure and Heart Rate in Rats with Aortic-Banding | | | | | | | | |
|---|---|---|---|---|---|---|---|---|
| Group | No. of animals | Body weight (g) | Systolic pressure (mmHg) | Heart rate (beats/min) | Left ventricle weight (mg) | Left ventricle weight/body weight (mg/g) | Right ventricle weight (mg) | Right ventricle weight/ body weight (mg/g) |
| Sham | 19 | 282±2 | 146±3 | 450±7 | 565±10 | 2.01± 0.03 | 154±3 | 0.54± 0.01 |
| Control | 20 | 269±4 | 188±5** | 470±10 | 628± 12** | 2.34± 0.07** | 149±4 | 0.56± 0.02 |
| ANP | 12 | 283±3 | 188±8** | 465±7 | 592±10 | 2.09± 0.04# | 147±4 | 0.52± 0.02 |

Sham surgery was performed on animals of the sham group, while abdominal aorta constriction surgery was performed on animals of the control and ANP groups. Continuous intravenous infusion of 5% glucose at 2.5 µl/min for animals of the sham and control groups, or α-hANP at 0.1 µg/kg/min for animals of the ANP group, was performed for 1 week starting immediately after surgery.

Each value represents the mean ± standard error.

Single and double asterisks (*, **) indicate the presence of a significant difference with the sham group at p<0.05 or p<0.01, respectively, as determined by one-way analysis of variance (ANOVA).

Number marks (#) indicate the presence of a significant difference with the control group at p<0.05 as determined by ANOVA analysis.

As shown in Table 1, there were no significant differences observed in body weight and heart rate among the sham, control and ANP groups one week after aortic banding and the start of drug administration. In the control group, a significant increase in systolic blood pressure as compared with the sham group was observed due to increased vascular resistance resulting from aortic banding, and ANP did not affect blood pressure. On the other hand, although a significant increase in left ventricle weight as compared with the sham group was observed in the control group, increasing significantly from 2.01±0.03 to 2.34±0.07, in the ANP group, there was little increase in left ventricle weight, while the ratio of left ventricle weight to body weight (mg/g) was significantly less than the control group at 2.09±0.04. There were no differences in the ratio of right ventricle weight/body weight among any of the groups.

Plasma α-hANP concentration on day 7 of administration in the ANP group was 502±72 pg/ml (approx. 0.17 nM). In addition, the plasma concentration of cGMP, a second messenger of intracellular signal transduction of ANP, was 5.3±0.4 pmole/ml in the ANP group, thus indicating higher values than those of the sham and control groups (2.0±0.3 and 3.4±0.5 pmole/ml, respectively), thereby confirming that ANP was infused continuously.

In addition, in contrast to intrinsic ANP concentration in plasma being 83±8 pg/ml in the sham group, it significantly increased to 139±19 pg/ml in the control group. This suggests that compensatory secretion of intrinsic ANP was increased in response to heart load induced by aortic banding.

On the other hand, intrinsic ANP concentration in the ANP group tended to exhibit a low value of 113±10 pg/ml in reflection of inhibition of the formation of hypertrophy.

In addition, in this study, the rats were housed in metabolic cages after the start of α-hANP administration and urine was collected every 24 hours to measure urine volume and urine sodium excretion. As shown in Fig. 1, there were no significant differences observed in urine volume or urine sodium excretion during the administration period among any of the groups, and diuretic effects produced by administration of ANP were not observed in this model.

In this manner, the formation of cardiac hypertrophy was shown to be inhibited by continuous intravenous infusion of ANP for 1 week. At this time, since there were no effects on body weight or right ventricle weight, the effect of ANP was considered to be specific for the left ventricle which is subjected to loading, and not the result of systemic metabolic disturbances or cytotoxicity. In addition, since diuretic and hypotensive effects of ANP were not observed in this model at the dose level (0.1 µg/kg/min) used in this study, ANP is suggested to have inhibited the onset of cardiac hypertrophy by acting directly on the heart.

### Example 2. Study of Reducing Effects of ANP on Cardiac Hypertrophy

Next, the potential for having reducing effects on cardiac hypertrophy was examined by continuously infusion of ANP after hypertrophy had already occurred according to the protocol indicated in Embodiment 1.

After cardiac hypertrophy had reached a steady state by allowing rats on which abdominal aortic banding or sham surgery had been performed to go untreated for 15 weeks, a silicon catheter was inserted into the jugular vein followed by continuous intravenous infusion of α-hANP at 0.1 µg/kg/min or 5% glucose at 2.5 µl/min for 3 weeks. Hemodynamics of the rats were measured under anesthesia 3 weeks after the start of administration, and the rats were sacrificed after collecting blood samples followed by measurement of heart weight. Measured values for heart weight and hemodynamics parameters at the end of drug administration in the sham, control and ANP groups are shown in Table 2.

**Table 2**

| Effects of Continuous Intravenous Infusion of ANP for 3 Weeks on Body Weight, Heart Weight, Blood Pressure and Heart Rate in Rats 15 Weeks After Abdominal Aortic-Banding | | | | | | | | |
|---|---|---|---|---|---|---|---|---|
| Group | No. of animals | Body weight (g) | Systolic pressure (mmHg) | Heart rate (beats/min) | Left ventricle weight (mg) | Left ventricle weight/body weight (mg/g) | Right ventricle weight (mg) | Right ventricle weight/body weight (mg/g) |
| Sham | 9 | 523±11 | 136±8 | 380±16 | 938±15 | 1.80± 0.04 | 217±6 | 0.42± 0.02 |
| Control | 9 | 492±10 | 214±8** | 428± 14** | 1171± 25** | 2.38± 0.05** | 214±6 | 0.44± 0.01 |
| ANP | 12 | 502±9 | 205±8** | 451±9** | 1056± 20**,## | 2.11± 0.06**, | 220±4 | 0.44± 0.01 |

Sham surgery was performed on animals of the sham group, while abdominal aortic banding was performed on animals of the control and ANP groups. Continuous intravenous infusion of 5% glucose at 2.5 µl/min for animals of the sham and control groups, or α-hANP at 0.1 µg/kg/min for animals of the ANP group, was performed for 3 weeks starting 15 weeks after surgery.

Each value represents the mean ± standard error.

Single and double asterisks (*, **) indicate the presence of a significant difference with the sham group at p<0.05 or p<0.01, respectively, as determined by one-way analysis of variance (ANOVA).

Number marks (##) indicate the presence of a significant difference with the control group at p<0.01 as determined by one-way analysis of variance (ANOVA).

As is shown in Table 2, there were no significant differences in body weight among the groups. In the control group, a significant increase in systolic blood pressure as compared with the sham group was observed due to increased vascular resistance resulting from aortic banding, and effects of ANP on blood pressure were not observed. Heart rate was significantly increased in the control and ANP groups as compared with the sham group.

Left ventricle weight and the ratio of left ventricle weight/body weight used as an indicator of cardiac hypertrophy were significantly increased in the control group (2.36±0.05) and ANP group (2.11±0.06) as compared with the sham group (1.80±0.04), and although cardiac hypertrophy was observed, significantly lower values were exhibited by the ANP group as compared with the control group, thus indicating a reduction in hypertrophy. There were no differences in the ratio of right ventricle weight/body weight in any of the groups, thus indicating that the heart weight lowering effect of ANP was specific for the hypertrophied left ventricle.

Furthermore, since the left ventricle weight 15 weeks after surgery when drug administration was started was 937±23 mg in the sham group and 1199±28 mg in the control group (n = 3 for each), there was a prominent increase in left ventricle weight in the control group as compared with the sham group, thus indicating the formation of cardiac hypertrophy. Since there were no differences in heart weight at this time between that at completion of drug administration (after 18 weeks) [sham group: 938±15 mg; control group: 1171±25 mg (n = 9 for each)], this indicated that cardiac hypertrophy due to pressure loading had been completed and reached a steady state by 15 weeks after surgery, and that there was very little additional progress of hypertrophy during the drug administration period (15 to 18 weeks after surgery). Thus, the fact that the ratio of left ventricle weight/body weight decreased by the administration of ANP starting 15 weeks after surgery suggests that ANP is effective for the reduction of hypertrophy that has already formed.

At this time, intrinsic rat ANP concentration was significantly increased in the control group (126±9 pg/ml) as compared with the sham group (70±5 pg/ml), and tended to be lower (105±18 pg/ml) in the ANP group. In addition, plasma α-hANP concentration at completion of administration in the ANP group was 426±53 pg/ml (approx. 0.14 nM).

In this manner, ANP demonstrated effects that not only prevent the formation of cardiac hypertrophy, but also cause a reduction in hypertrophy that has already formed.

### Example 3. Study of Cardiac hypertrophy and Pulmonary Congestion Alleviating Effects of ANP

Continuous intravenous infusion of α-hANP at 0.1 µg/kg/min or 5% glucose at 2.5 µl/min was performed in rats starting 2 weeks after performing arteriovenous shunt surgery or sham surgery to examine the effects of ANP on cardiac hypertrophy and pulmonary congestion. Measured values for body weight and hemodynamics parameters at the end of drug administration in the sham, control and ANP groups are shown in Table 3.

**Table 3**

| Effects of Continuous Intravenous Infusion of ANP for 2 Weeks on Body Weight, Blood Pressure, Heart Rate and Right Atrial Pressure in Rats 2 Weeks After Arteriovenous Shunt Surgery | | | | | |
|---|---|---|---|---|---|
| Group | No. of animals | Body weight (g) | Systolic pressure (mmHg) | Heart rate (beats/min) | Right atrial pressure |
| Sham | 6 | 375±10 | 148±7 | 434±21 | 7.3±0.5 |
| Control | 6 | 373±12 | 145±6 | 436±18 | 13.6±2.6** |
| ANP | 6 | 369±9 | 143±4 | 460±14 | 12.6±0.7* |

Sham surgery was performed on animals of the sham group, while abdominal arteriovenous shunt surgery was performed on animals of the control and ANP groups. Continuous intravenous infusion of 5% glucose at 2.5 µl/min for animals of the sham and control groups, or α-hANP at 0.1 µg/kg/min for animals of the ANP group, was performed for 2 weeks starting 2 weeks after surgery.

Each value represents the mean ± standard error.

Single and double asterisks (*, **) indicate the presence of a significant difference with the sham group at p<0.05 or p<0.01, respectively, as determined by ANOVA analysis.

As shown in Table 3, there were no significant differences in body weight among any of the groups. There were also no differences in mean blood pressure and heart rate among the groups. In the control group, a significant increase in right atrial pressure was observed in comparison with the sham group due to increased volume loading induced by arteriovenous shunt. Right atrial pressure was also increased in the ANP group similar to the control group, and effects of ANP on hemodynamics were not observed.

In addition, the ratios (mg/g) of right atrium, left atrium, right ventricle and left ventricle weights to body weight, which served as indicators of cardiac hypertrophy, are shown in Fig. 2. Values were significantly higher in the control group as compared with the sham group at all sites, thus indicating prominent formation of hypertrophy. On the other hand, the ratios of right atrium, left atrium, right ventricle and left ventricle weights to body weight were all significantly lower in the ANP group as compared with the control group, thereby indicating that ANP inhibited the formation of cardiac hypertrophy.

Moreover, the ratio of lung weight to body weight (mg/g) and hematocrit (%), which served as indicators of pulmonary congestion, are shown in Fig. 3. Although the control group exhibited significantly higher values than the sham group with respect to the ratio of lung weight to body weight (sham group: 4.02±0.13; control group: 4.81±0.17], in the ANP group, values decreased to about the same level as the sham group (4.06±0.25), with ANP observed to ameliorate pulmonary congestion. With respect to hematocrit values as well, since the control group exhibited lower values than the sham group, increased blood volume, namely a trend towards systemic congestion, was suggested, while values in the ANP group were improved.

Since ANP did not affect blood pressure or right atrial pressure in this model as well at the dose levels used in this study (0.1 µg/kg/min), ANP is believed to have inhibited the onset of hypertrophy by acting directly on the heart. In contrast to intrinsic rat ANP concentration at this time having been 43±11 pg/ml in the sham group, it increased remarkably to 854±265 pg/ml in the control group, while decreasing significantly in the ANP group (419±202 pg/ml). These results support the results of cardiac hypertrophy formation by shunt surgery as well as inhibition of cardiac hypertrophy by administration of ANP. In addition, the plasma α-hANP concentration at completion of administration in the ANP group was 329±113 pg/ml (approx. 0.11 nM).

Furthermore, although a slight increase in the ratio of right atrium weight to body weight was observed in the control group as compared with the sham group at 2 weeks after surgery when drug administration was started (sham group: 0.10±0.01 (n = 6), control group: 0.13±0.004 (n = 8)), there were no differences between the groups with respect to the ratios of left atrium, right ventricle, left ventricle and lung weights to body weight, while plasma rat ANP concentration only tended to be slightly increased (sham group: 34±3 pg/ml (n = 6), control group: 47±8 pg/ml (n = 8)). In this manner, there was hardly any occurrence of a disease state at 2 weeks after abdominal aorta arteriovenous shunt surgery, while the disease state is believed to have progressed from 2 weeks to 4 weeks after surgery. Thus, it was suggested that administration of ANP from 2 weeks to 4 weeks after surgery inhibited the formation of cardiac hypertrophy and pulmonary congestion.

As has been described above, ANP was shown to inhibit not only hypertensive left ventricular hypertrophy induced by pressure loading, but also the formation of cardiac hypertrophy induced by volume loading. In addition, ANP was also shown to be effective not only in terms of the onset of cardiac hypertrophy, but also in terms of ameliorating pulmonary congestion.

### Industrial Applicability

The present invention has proven that a composition having for its active ingredient a substance that acts on the natriuretic peptide receptor, GC-A, and is able to stimulate production of cGMP is effective in preventing cardiac hypertrophy involved in the onset and progress of heart diseases such as chronic heart failure. In the case of using ANP as the active ingredient in particular, the fact that ANP not only inhibited the formation of hypertrophy but also exhibited reducing effects in a pressure-loaded hypertrophy model suggests that it is therapeutically effective and clinically useful in the case of using in patients in which cardiac hypertrophy has already occurred.

In addition, since ANP exhibited inhibitory effects on cardiac hypertrophy as well as pulmonary congestion ameliorating effects in a volume-loaded hypertrophy model, it was suggested that ANP is also effective in the case of using in patients with pulmonary congestion, a major symptom of chronic heart failure and a cause of dyspnea. Moreover, since ANP exhibits inhibitory effects on hypertrophy and pulmonary congestion at dose levels at which it does not affect blood pressure, heart rate or urine volume, it has few adverse side effects in terms of hemodynamics and is considered to be able to be used safely.

Based on these results, ANP was strongly suggested to be able to improve cardiac hypertrophy based on high blood pressure, valvopathy or myocardial infarction, as well as pulmonary congestion that occurs as a result of cardiac dysfunction. Cardiac hypertrophy is itself an independent risk factor for ischemic heart disease, arrhythmia and chronic heart failure, while chronic heart failure in particular is a disease having a high mortality rate and poor prognosis. Accordingly, under the present circumstances in which there is an extremely dire need for a new drug that is effective in improving cardiac hypertrophy and ameliorating the load on the heart, the pharmaceutical composition for treatment of heart disease based on cardiac hypertrophy as claimed in the present invention is extremely useful.

SEQUENCE LISTING
Sequence No.: 1
Sequence length: 28
Sequence type: Amino acid
Topology: Linear
Molecular type: Peptide
Sequence
Ser Leu Arg Arg Ser Ser Cys Phe Gly Gly Arg Met Asp Arg Ile Gly
1               5                   10                  15
Ala Gln Ser Gly Leu Gly Cys Asn Ser Phe Arg Tyr
            20                  25
Sequence No.: 2
Sequence length: 28
Sequence type: Amino acid
Topology: Linear
Molecular type: Peptide
Sequence
Ser Leu Arg Arg Ser Ser Cys Phe Gly Gly Arg Ile Asp Arg Ile Gly
1               5                   10                  15
Ala Gln Ser Gly Leu Gly Cys Asn Ser Phe Arg Tyr
            20                  25
Sequence No.: 3
Sequence length: 22
Sequence type: Amino acid
Topology: Linear
Molecular type: Peptide
Sequence
Cys Phe Gly Gly Arg Met Asp Arg Ile Gly Ala Gln Ser Gly Leu Gly
1               5                   10                  15
Cys Asn Ser Phe Arg Tyr
            20
Sequence No.: 4
Sequence length: 32
Sequence type: Amino acid
Topology: Linear
Molecular type: Peptide
Sequence

Ser Pro Lys Met Val Gln Gly Ser Gly Cys Phe Gly Arg Lys Met Asp
1               5                   10                  15
Arg Ile Ser Ser Ser Ser Gly Leu Gly Cys Lys Val Leu Arg Arg His
            20                  25                  30
Sequence No.: 5
Sequence length: 24
Sequence type: Amino acid
Topology: Linear
Molecular type: Peptide
Sequence
Ser Ser Asp Cys Phe Gly Ser Arg Ile Asp Arg Ile Gly Ala Gln Ser
1               5                   10                  15
Gly Met Gly Cys Gly Arg Arg Phe
            20
Sequence No.: 6
Sequence length: 32
Sequence type: Amino acid
Topology: Linear
Molecular type: Peptide
Sequence
Ser Pro Lys Thr Met Arg Asp Ser Gly Cys Phe Gly Arg Arg Leu Asp
1               5                   10                  15
Arg Ile Gly Ser Leu Ser Gly Leu Gly Cys Asn Val Leu Arg Arg Tyr
            20                  25                  30
Sequence No.: 7
Sequence length: 45
Sequence type:
No. of chains:
Topology: Linear
Molecular type:
Sequence
Ser Gln Asp Ser Ala Phe Arg Ile Gln Glu Arg Leu Arg Asn Ser Lys
1               5                   10                  15
Met Ala His Ser Ser Ser Cys Phe Gly Gln Lys Ile Asp Arg Ile Gly
            20                  25                  30
Ala Val Ser Arg Leu Gly Cys Asp Gly Leu Arg Leu Phe
        35                  40                  45

Sequence No.: 8
Sequence length: 29
Sequence type: Amino acid
Topology: Linear
Molecular type: Peptide
Sequence
Met Met Arg Asp Ser Gly Cys Phe Gly Arg Arg Ile Asp Arg Ile Gly
1               5                   10                  15
Ser Leu Ser Gly Met Gly Cys Asn Gly Ser Arg Lys Asn
            20                  25

## Claims

1. A pharmaceutical composition for treatment of heart disease based on cardiac hypertrophy comprising as its active ingredient a substance that acts on the natriuretic peptide receptor, guanilyl cyclase A, and is able to accelerate production of cyclic guanosine monophosphate.

2. A pharmaceutical composition as set forth in claim 1 wherein the heart disease based on cardiac hypertrophy is chronic heart failure.

3. A pharmaceutical composition as set forth in claim 1 wherein the substance that acts on the natriuretic peptide receptor, guanilyl cyclase A, and is able to accelerate production of cyclic guanosine monophosphate is natriuretic peptide.

4. A pharmaceutical composition as set forth in claim 3 wherein natriuretic peptide is atrial natriuretic peptide.

5. A pharmaceutical composition as set forth in claim 3 wherein natriuretic peptide is brain natriuretic peptide.

6. The use of a substance that acts on the natriuretic peptide receptor, guanylyl cyclase A, and is able to accelerate production of cyclic guanosine monophosphate, for the production of a pharmaceutical composition for treatment of heart disease based on cardiac hypertrophy.

7. The use as set forth in claim 6 wherein the heart disease based on cardiac hypertrophy is chronic heart failure.

8. The use as set forth in claim 10 wherein the substance that acts on the natriuretic peptide receptor, guanylyl cyclase A, and is able to accelerate production of cyclic guanosine monophosphate is natriuretic peptide.

9. The use as set forth in claim 8 wherein natriuretic peptide is atrial natriuretic peptide.

10. The use as set forth in claim 13 wherein natriuretic peptide is brain natriuretic peptide.
